# EUROPEAN PATENT APPLICATION

(11) **EP 4 492 384 A1**
(43) Date of publication of application: **15.01.2025**
(21) Application number: 24183434.0
(22) Date of filing: 20.06.2024
(51) Int. Cl.: G16B 15/30, G16C 20/10, G16C 20/50, G16C 20/30, G16C 20/70

(54) **SYSTEMS AND METHODS FOR SYNTHESIS-AWARE GENERATION OF PROPERTY OPTIMIZED SMALL MOLECULES**

(30) Priority: 11.07.2023 IN 202321046650
(71) Applicant: Tata Consultancy Services Limited, Maharashtra (IN)
(72) Inventor: KRISHNAN, SOWMYA RAMASWAMY, 600113 Chennai, Tamil Nadu (IN); ROY, ARIJIT, 500034 Hyderabad, Telangana (IN); BUNG, NAVNEET, 500034 Hyderabad, Telangana (IN); SRINIVASAN, RAJGOPAL, 500034 Hyderabad, Telangana (IN)
(74) Representative: Goddar, Heinz J.

(57) **Abstract**

Deep learning-based generative models have improved the exploration of chemical space in small molecule drug discovery. Although thousands of novel small molecules can be generated with such models, synthesizing them still remains a challenging task. In literature, several methods have been proposed to predict the synthetic route of a target molecule by working backwards to find the most suitable starting reactants (retrosynthesis). While retrosynthesis is shown to be successful, for novel molecules it is often difficult to find the synthesis path. System and method of the present disclosure generate molecules along with its synthesis route and also provide an insight into the interactions in the active site of target protein, using graph convolution networks (GCNs) and Monte Carlo tree search (MCTS). A target-specific bioactivity prediction model is used as the scoring function to navigate the MCTS search space efficiently.

## Description

### CROSS-REFERENCE TO RELATED APPLICATIONS AND PRIORITY

The present application claims priority to Indian application no. 202321046650, filed on July 11, 2023.

### TECHNICAL FIELD

The disclosure herein generally relates to generation of small molecule using forward synthesis techniques, and, more particularly, to systems and methods for synthesis-aware generation of property optimized small molecules.

### BACKGROUND

Deep learning-based generative models have improved the exploration of chemical space in small molecule drug discovery. Although thousands of novel small molecules can be generated with such models, synthesizing them still remains a challenging task. In literature, several methods have been proposed to predict the synthetic route of a target molecule by working backwards to find the most suitable starting reactants (retrosynthesis). While retrosynthesis is shown to be successful, for novel molecules it is often difficult to find the synthesis path.

### SUMMARY

Embodiments of the present disclosure present technological improvements as solutions to one or more of the above-mentioned technical problems recognized by the inventors in conventional systems.

For example, in one aspect, there is provided a processor implemented method for synthesis-aware generation of property optimized small molecules. The method comprises (i) selecting, via one or more hardware processors, a first molecular fragment from a plurality of molecular fragments comprised in a fragment library; (ii) performing, at least one docking technique via the one or more hardware processors, on the first molecular fragment to obtain a first docking output; (iii) computing, via the one or more hardware processors, a first bioactivity value of the first molecular fragment using a drug-target affinity (DTA) model based on the first docking output, wherein a search tree is populated with the first bioactivity value for the first molecular fragment; (iv) determining, via the one or more hardware processors, a plurality of reaction templates pertaining to the first molecular fragment using a trained reaction template selection model; (v) performing, via the one or more hardware processors, for each reaction template amongst the plurality of reaction templates based on a type of a reaction template, one of: obtaining a reaction intermediate based on the first molecular fragment; and selecting a second molecular fragment using a trained molecular fragment selection policy and obtaining the reaction intermediate, to obtain one or more reaction intermediates; (vi) performing, the at least one docking technique via the one or more hardware processors, on the one or more reaction intermediates to obtain a second docking output; (vii) computing, via the one or more hardware processors, a second bioactivity value of the one or more reaction intermediates using the DTA model based on the second docking output; (viii) selecting, via the one or more hardware processors, at least one reaction intermediate amongst one or more reaction intermediates based on the second bioactivity value, wherein the at least one reaction intermediate serves as a child node of the first molecular fragment; (ix) iteratively performing, via the one or more hardware processors, a simulation on the search tree by repeating the steps of (iv) through (viii), using the child node, to obtain a best child node, wherein the child node is appended to the first molecular fragment along with the first bioactivity value, or the best child node based on a previous iteration, until at least one of (i) a bioactivity value of a selected child node is greater than a predefined bioactivity threshold and (ii) a depth of the search tree is greater than a maximum depth allowed; and (x) generating, via the one or more hardware processors, a molecule with a synthesis route based on a path selected using one or more best child nodes from the search tree, wherein the molecule is used as a promising candidate during subsequent drug design and development.

In an embodiment of the present disclosure, the at least one docking technique is a tethered docking technique or an untethered docking technique.

In an embodiment of the present disclosure, the at least one docking technique is a tethered docking technique when the first molecular fragment is selected from a result obtained from a crystallographic screening.

In an embodiment of the present disclosure, the at least one docking technique is an untethered docking technique when the first molecular fragment is randomly selected.

In an embodiment of the present disclosure, the least one reaction intermediate comprises a highest bioactivity value.

In an embodiment of the present disclosure, the trained molecular fragment selection policy is configured to select the second molecular fragment by: predicting a property vector of one or more candidate second molecular fragments from at least one fragment library obtained from at least one source; and selecting the second molecular fragment from the fragment library based a comparison of the property vector of the one or more candidate second molecular fragments and an associated property vector of each molecular fragment comprised in the at least one fragment library.

In an embodiment of the present disclosure, the type of the reaction template comprises at least one of a unimolecular reaction template, and a bimolecular reaction template.

In another aspect, there is provided a processor implemented system for synthesis-aware generation of property optimized small molecules. The system comprises: a memory storing instructions; one or more communication interfaces; and one or more hardware processors coupled to the memory via the one or more communication interfaces, wherein the one or more hardware processors are configured by the instructions to: (i) select a first molecular fragment from a plurality of molecular fragments comprised in a fragment library; (ii) perform, at least one docking technique, on the first molecular fragment to obtain a first docking output; (iii) compute a first bioactivity value of the first molecular fragment using a drug-target affinity (DTA) model based on the first docking output, wherein a search tree is populated with the first bioactivity value for the first molecular fragment; (iv) determine a plurality of reaction templates pertaining to the first molecular fragment using a trained reaction template selection model; (v) perform for each reaction template amongst the plurality of reaction templates based on a type of a reaction template, one of: obtaining a reaction intermediate based on the first molecular fragment; and selecting a second molecular fragment using a trained molecular fragment selection policy and obtaining the reaction intermediate, to obtain one or more reaction intermediates; (vi) perform, the at least one docking technique, on the one or more reaction intermediates to obtain a second docking output; (vii) compute a second bioactivity value of the one or more reaction intermediates using the DTA model based on the second docking output; (viii) select at least one reaction intermediate amongst one or more reaction intermediates based on the second bioactivity value, wherein the at least one reaction intermediate serves as a child node of the first molecular fragment; (ix) iteratively perform a simulation on the search tree by repeating the steps of (iv) through (viii), using the child node, to obtain a best child node, wherein the child node is appended to the first molecular fragment along with the first bioactivity value, or the best child node based on a previous iteration, until at least one of (a) a bioactivity value of a selected child node is greater than a predefined bioactivity threshold and (b) a depth of the search tree is greater than a maximum depth allowed; and (x) generate a molecule with a synthesis route based on a path selected using one or more best child nodes from the search tree, wherein the molecule is used as promising candidate during subsequent drug design and development.

In an embodiment of the present disclosure, the at least one docking technique is a tethered docking technique or an untethered docking technique.

In an embodiment of the present disclosure, the at least one docking technique is a tethered docking technique when the first molecular fragment is selected from a result obtained from a crystallographic screening.

In an embodiment of the present disclosure, the at least one docking technique is an untethered docking technique when the first molecular fragment is randomly selected.

In an embodiment of the present disclosure, the at least one reaction intermediate comprises a highest bioactivity value.

In an embodiment of the present disclosure, the trained molecular fragment selection policy is configured to select the second molecular fragment by: predicting a property vector of one or more candidate second molecular fragments from at least one fragment library obtained from at least one source; and selecting the second molecular fragment from the fragment library based a comparison of the property vector of the one or more candidate second molecular fragments and an associated property vector of each molecular fragment comprised in the at least one fragment library.

In an embodiment of the present disclosure, the type of the reaction template comprises at least one of a unimolecular reaction template, and a bimolecular reaction template.

In yet another aspect, there are provided one or more non-transitory machine-readable information storage mediums comprising one or more instructions which when executed by one or more hardware processors cause synthesis-aware generation of property optimized small molecules by (i) selecting a first molecular fragment from a plurality of molecular fragments comprised in a fragment library; (ii) performing, at least one docking technique, on the first molecular fragment to obtain a first docking output; (iii) computing a first bioactivity value of the first molecular fragment using a drug-target affinity (DTA) model based on the first docking output, wherein a search tree is populated with the first bioactivity value for the first molecular fragment; (iv) determining a plurality of reaction templates pertaining to the first molecular fragment using a trained reaction template selection model; (v) performing, for each reaction template amongst the plurality of reaction templates based on a type of a reaction template, one of: obtaining a reaction intermediate based on the first molecular fragment; and selecting a second molecular fragment using a trained molecular fragment selection policy and obtaining the reaction intermediate, to obtain one or more reaction intermediates; (vi) performing, the at least one docking technique, on the one or more reaction intermediates to obtain a second docking output; (vii) computing a second bioactivity value of the one or more reaction intermediates using the DTA model based on the second docking output; (viii) selecting at least one reaction intermediate amongst one or more reaction intermediates based on the second bioactivity value, wherein the at least one reaction intermediate serves as a child node of the first molecular fragment; (ix) iteratively performing a simulation on the search tree by repeating the steps of (iv) through (viii), using the child node, to obtain a best child node, wherein the child node is appended to the first molecular fragment along with the first bioactivity value, or the best child node based on a previous iteration, until at least one of (a) a bioactivity value of a selected child node is greater than a predefined bioactivity threshold and (b) a depth of the search tree is greater than a maximum depth allowed; and (x) generating a molecule with a synthesis route based on a path selected using one or more best child nodes from the search tree, wherein the molecule is used as promising candidate during subsequent drug design and development.

In an embodiment of the present disclosure, the at least one docking technique is a tethered docking technique or an untethered docking technique.

In an embodiment of the present disclosure, the at least one docking technique is a tethered docking technique when the first molecular fragment is selected from a result obtained from a crystallographic screening.

In an embodiment of the present disclosure, the at least one docking technique is an untethered docking technique when the first molecular fragment is randomly selected.

In an embodiment of the present disclosure, the at least one reaction intermediate comprises a highest bioactivity value.

In an embodiment of the present disclosure, the trained molecular fragment selection policy is configured to select the second molecular fragment by: predicting a property vector of one or more candidate second molecular fragments from at least one fragment library obtained from at least one source; and selecting the second molecular fragment from the fragment library based a comparison of the property vector of the one or more candidate second molecular fragments and an associated property vector of each molecular fragment comprised in the at least one fragment library.

In an embodiment of the present disclosure, the type of the reaction template comprises at least one of a unimolecular reaction template, and a bimolecular reaction template.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 depicts an exemplary system for synthesis-aware generation of property optimized small molecules, in accordance with an embodiment of the present disclosure.
FIG. 2 depicts an exemplary high level block diagram of the system of FIG. 1 for synthesis-aware generation of property optimized small molecules, in accordance with an embodiment of the present disclosure.
FIG. 3A and FIG. 3B (collectively referred to as FIG. 3) depict an exemplary flow chart illustrating a method for synthesis-aware generation of property optimized small molecules, using the systems of FIG. 1-2, in accordance with an embodiment of the present disclosure.
FIG. 4 depicts crystallographic fragments and their corresponding scaffolds chosen for tethered docking based MCTS experiments from protein data bank (PDB) structures (a) 3NX8; (b) 3OOG; (c) 5BX6; (d) 5BX7; (e) 5N3Q, in accordance with an embodiment of the present disclosure.
FIG. 5 depicts a complete forward synthesis route predicted by Monte Carlo Tree Search (MCTS) search tree with the tethered docking technique for run 1 of molecular fragment (PDB ID: 3NX8), in accordance with an embodiment of the present disclosure.
FIG. 6 depicts the complete forward synthesis route predicted by Monte Carlo Tree Search (MCTS) search tree with the tethered docking technique for run 1 of molecular fragment (PDB ID: 3OOG), in accordance with an embodiment of the present disclosure.
FIG. 7 depicts the complete forward synthesis route predicted by Monte Carlo Tree Search (MCTS) search tree with the tethered docking technique for run 1 of molecular fragment (PDB ID: 5BX6), in accordance with an embodiment of the present disclosure.
FIG. 8 depicts the complete forward synthesis route predicted by Monte Carlo Tree Search (MCTS) search tree with the tethered docking technique for run 1 of molecular fragment (PDB ID: 5BX7), in accordance with an embodiment of the present disclosure.
FIG. 9 depicts a representation of how molecules are anchored to the binding site based on the scaffold present in a crystallographic (molecular) fragment, in an accordance with an embodiment of the present disclosure.
FIG. 10 depicts changes observed in an interaction profile of a small molecule (run 1) at a PKA binding site, over the course of the tethered docking based MCTS calculations, in accordance with an embodiment of the present disclosure.
FIG. 11 depicts an expansion step from the first molecular fragment for selection of at least one reaction intermediate amongst one or more reaction intermediates, in accordance with an embodiment of the present disclosure.
FIG. 12 depicts a complete MCTS search tree showing a termination of the MCTS search tree process upon generation of a child node with reward greater than the necessary reward threshold (in this case, 8.5), in accordance with an embodiment of the present disclosure.
FIG. 13 depicts a histogram illustrating (a) time distribution of the MCTS runs for the building blocks enriched in PKA inactives, wherein an average time taken is 450 mins per run, and (b) distribution of a predicted log-scale bioactivity against PKA for final products obtained from MCTS, in accordance with an embodiment of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Deep learning models have remodeled the space of drug discovery by enabling generic and specific molecule generation, property prediction and on-the-fly molecular optimization in the past couple years. The unimaginable pace of these models coupled with their state-of-the-art predictive abilities, has led to a tremendous acceleration of the early-stage molecule discovery process. Several testaments to the success of deep neural networks in identifying clinically potent and efficacious small molecules have also emerged. Although the extent of chemical space currently explored by deep generative models remains unquantified, it is safer to say that they can explore a sufficiently diverse and relevant space of potentially novel small molecules with the aid of techniques such as Transfer Learning (TL) and Reinforcement Learning (RL). With the models currently available in literature, it is technically possible to design drug-like small molecules for any given target protein of interest using either the protein sequence or structure or a set of known inhibitors identified *apriori.* However, further experimental validation of the designed small molecules requires that the molecule be easily synthesizable and stable in the physiological conditions for mass production and clinical testing.

Several studies have tried to address the problem of synthetic accessibility of generated small molecules through two primary methods: quantification of synthesizability with metrics and *in silico* prediction of the probable synthesis route(s) for the molecule. Synthesizability has been traditionally quantified in terms of the contribution of fragments toward the overall complexity of the molecular structure. These metrics are often termed as synthetic accessibility scores which include, SAS, SCScore, RAscore, SYBA, GASA, and CMPNN. While SAS and SCscore follow the traditional approach to measure synthesizability, RAscore, SYBA, GASA and CMPNN utilize machine learning and deep learning models, with a varied set of molecular features underlying each model, to predict synthesizability. Based on a recent analysis it is evident that these metrics need to be finetuned according to the target model for synthetic route prediction. This suggests that the generalizability of these metrics needs to be carefully analyzed to discriminate molecules according to their degree of synthesizability. Several examples of molecules with conflicting synthesizability predictions have also been reported earlier, suggesting the need for a community-level perspective to emerge on this problem, to improve existing metrics and reach consensus.

On the other hand, the problem of synthetic route prediction has been approached vigorously, resulting in multiple approaches for both forward and retrosynthetic route prediction. All existing approaches can be categorized into either reaction template-based or template-free methods. Template-dependent approaches require a set of expertly curated chemical reaction rules, which generalize transformations from reactants to products. Template-independent approaches try to learn these rules from a large corpus of reactions in organic chemistry, therefore limiting their generalization capability to novel reactant datasets. Existing template-dependent methods explicitly limit the reactions to single-step or two-step synthesis problems to avoid traversing the highly diverse fragment space available. Two of the existing methods have enforced generation of molecules which are similar to existing approved drugs or known synthesizable small molecules, thereby significantly restricting the chemical space exploration by the model. While some approaches have tried to overcome this by allowing user-defined starting reactants, the molecules are again generated atom-by-atom through policy learning, which has been shown before to reduce chemical space exploration in previous studies. Only one of the existing approaches has tried to provide a structure-level perspective of the evolution of the molecule and its interactions with the binding site residues through 3D conformation generation. Although the coordinate space of the generation process was anchored to the binding site, the lack of minimization and analysis of short contacts post-generation leads to incomplete validation of the generated conformations.

To address the above challenges, present disclosure provides systems and methods that implement a synthesis-aware generative model which performs template-based forward synthesis route prediction and molecule generation simultaneously using the Monte Carlo Tree Search (MCTS) method. More specifically, the systems and methods of the present disclosure utilize the MCTS method for forward synthesis route prediction. Rewards for each iteration of MCTS are computed using a drug-target affinity (DTA) model dependent on the docking pose of the generated reaction intermediates at the binding site of the target protein of interest. The synthesis-aware generative model utilizes a tethered docking approach wherein, crystallographic fragments can be provided as the starting point for molecule generation, and the corresponding fragment coordinates in the crystal structure can be used as the seed pose for docking. By ensuring that the seed structure is docked in a near-identical fashion between successive iterations of the MCTS method, the results could be interpreted in terms of the magnitude of improvement in predicted bioactivity with the incremental growth of the molecule within the binding site. In this way, the synthesis-aware generative model is designed to consider any available information regarding the protein structure while designing molecules and predict their forward synthesis route using libraries of globally purchasable reactants. The application of the method of the present disclosure is showcased through generation of molecules specific to the human cAMP-dependent protein kinase A (PKA).

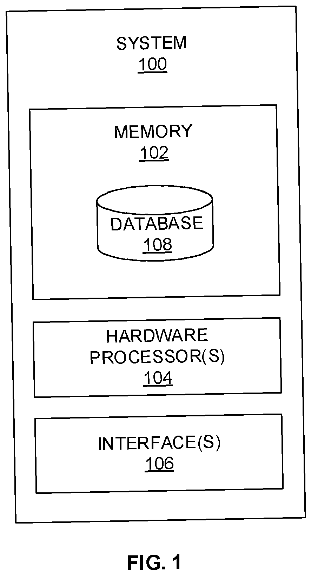

Referring now to the drawings, and more particularly to FIGS. 1 through 13, where similar reference characters denote corresponding features consistently throughout the figures, there are shown preferred embodiments and these embodiments are described in the context of the following exemplary system and/or method.

FIG. 1 depicts an exemplary system 100 for synthesis-aware generation of property optimized small molecules, in accordance with an embodiment of the present disclosure. The system 100 may also be referred to as molecule generation system and interchangeably used herein. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108 comprises molecular fragments, fragments library, docking outputs, bioactivity value of molecular fragments, a plurality of reaction templates, one or more reaction intermediates, one or more best child nodes, molecules with an associated synthesis route, and information associated therebetween. The database 108 further comprises search tree that is populated with bioactivity values for the molecular fragments, and is updated as per the requirements, and the like. The memory 102 further comprises one or more docking techniques (e.g., tethered docking technique, untethered docking technique, and the like), Monte Carlo Tree Search (MCTS) method, and the like, which when executed by the one or more hardware processors 104 enable the system 100 to perform the method described herein. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

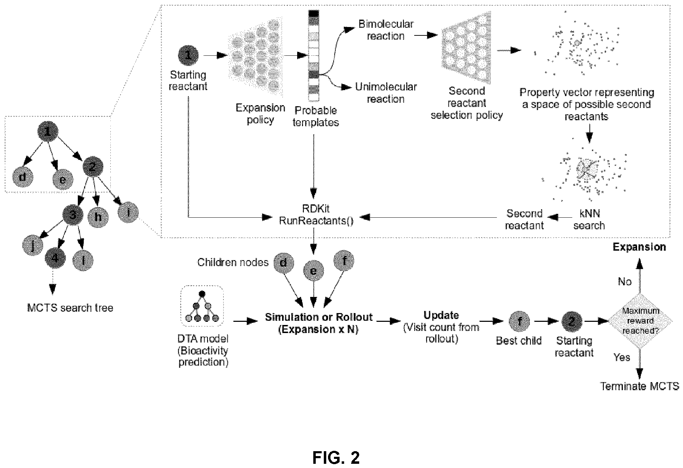

FIG. 2, with reference to FIG. 1, depicts an exemplary high level block diagram of the system 100 of FIG. 1 for synthesis-aware generation of property optimized small molecules, in accordance with an embodiment of the present disclosure.

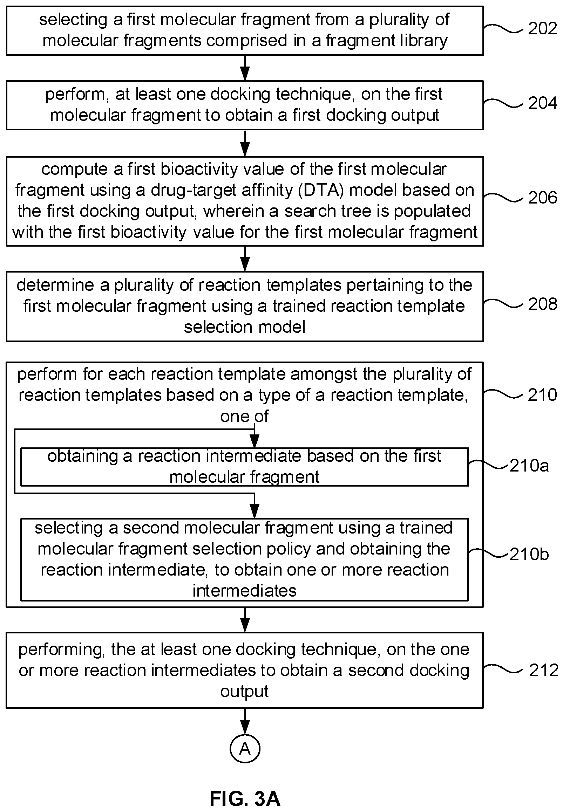

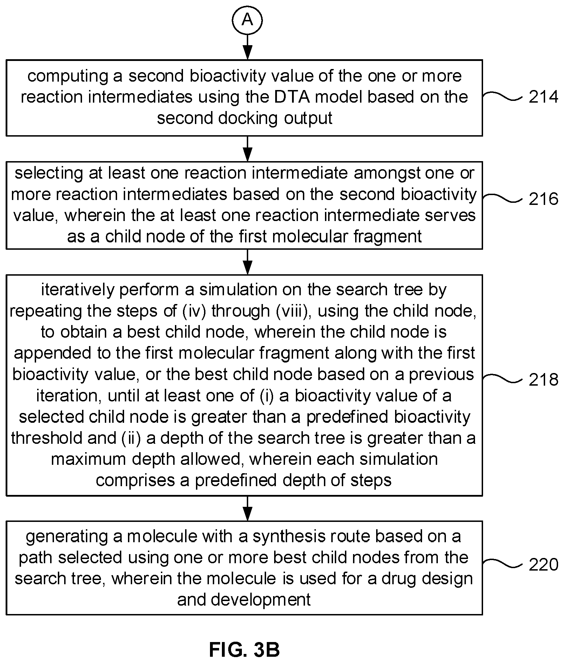

FIG. 3A and FIG. 3B (collectively referred to as FIG. 3), with reference to FIGS. 1-2, depict an exemplary flow chart illustrating a method for synthesis-aware generation of property optimized small molecules, using the systems 100 of FIG. 1-2, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, the block diagram of FIG. 2, and the flow diagram as depicted in FIG. 3. Although steps of the method of FIG. 3 including process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

At step 202 of the method of the present disclosure, the one or more hardware processors 104 select a first molecular fragment from a plurality of molecular fragments comprised in a fragment library. In the present disclosure, the first molecular fragment that is selected serves as a starting molecular fragment (or also referred to as first reactant and may be interchangeably used herein). The selection of the first molecular fragment may be better understood by way of following description.

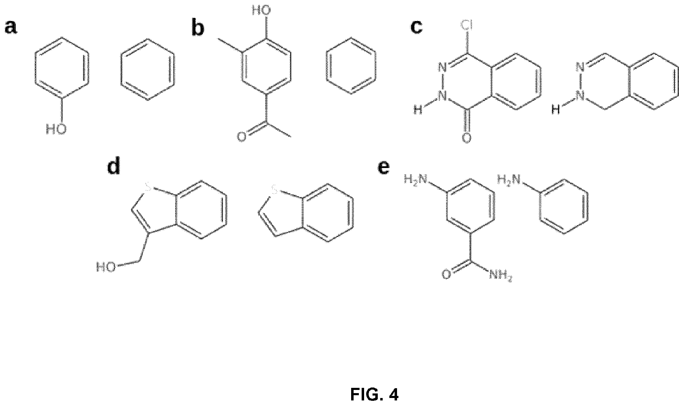

The dataset of the first molecular fragment was curated from an Enamine comprehensive building blocks database. Total 1,169,054 molecular fragment, part of the catalog was pre-processed using RDKit (e.g., an open-source toolkit for cheminformatics) to map each molecular fragment SMILES to their corresponding Enamine product identifier. The Enamine database was chosen to ensure that the starting molecular fragment corresponding to the reaction intermediates (also referred as intermediates and interchangeably used herein) identified as part of the forward synthesis route of any molecule are easily purchasable globally. Examples of molecular fragments are depicted in FIG. 4. More specifically, FIG. 4, with reference to FIGS. 1-3, depicts crystallographic fragments and their corresponding scaffolds chosen for tethered docking based MCTS experiments from protein databank id (a) 3NX8; (b) 3OOG; (c) 5BX6; (d) 5BX7; (e) 5N3Q, in accordance with an embodiment of the present disclosure.

At step 204 of the method of the present disclosure, the one or more hardware processors 104 perform, at least one docking technique via the one or more hardware processors, on the first molecular fragment to obtain a first docking output. The at least one docking technique is a tethered docking technique or an untethered docking technique, in an embodiment of the present disclosure. In the present disclosure, the at least one docking technique is the tethered docking technique when the first molecular fragment is selected from a result obtained from a crystallographic screening, and the at least one docking technique is the untethered docking technique when the first molecular fragment is randomly selected.

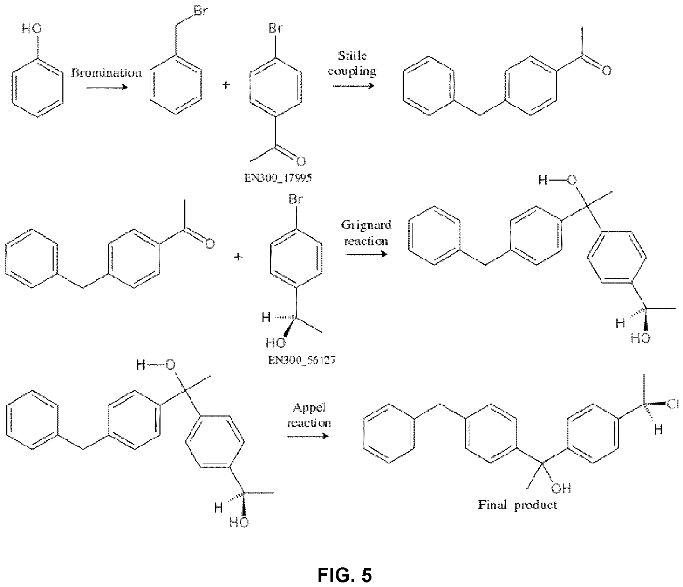

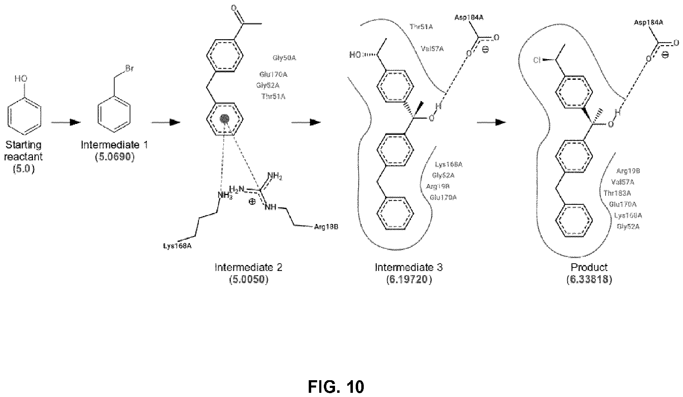

At step 206 of the method of the present disclosure, the one or more hardware processors 104 compute a first bioactivity value of the first molecular fragment using a drug-target affinity (DTA) model based on the first docking output. A search tree (also referred to as Monte Carlo Tree Search (MCTS) search tree and interchangeably used herein) is populated with the first bioactivity value for the first molecular fragment, in one embodiment of the present disclosure. The docking output from the docking step 204 is converted to an extended connectivity interaction fingerprint (ECIF), which is a one-dimensional vectorial representation of the protein-ligand interaction. This fingerprint is used as input to a trained DTA model specific to the PKA protein, to predict the bioactivity of the starting reactant or first molecular fragment. The obtained bioactivity value is used to update the reward and Q-value of the node (first molecular fragment) in the search tree. For instance, in case of molecular fragments shown in FIG. 5, docking poses obtained from rDock were used as input to the DTA model. The predicted log-scale bioactivity of the first fragment (e.g., refer FIG. 5(a)) was found to be 5.0 from the DTA model. This is also shown in FIG. 10. This information will be used to populate the empty search tree with the first molecular fragment (root node) and its bioactivity value (5.0). More specifically, FIG. 10, with reference to FIGS. 1 through 9, depicts changes observed in an interaction profile of a small molecule (run 1) at a PKA binding site, over the course of the tethered docking based MCTS calculations, in accordance with an embodiment of the present disclosure. The steps of 204 and 206 are better understood by way of following description:
To initiate the MCTS search tree, a starting reactant or the first molecular fragment is required. For the tethered docking technique use case, this first molecular fragment has been taken from a crystal structure and for the untethered docking technique use case, this first molecular fragment is taken from a fragment library that is a purchasable fragment library such as Enamine (from at least one source). The fragment selection can be random or can be based on existing inhibitors for a target protein. In the present disclosure, rDock program was used to perform docking technique on the first molecular fragment pertaining to the crystal structure of the target protein. For instance, in case of the molecular fragments shown in FIG. 4, the binding site of the molecular fragments in the PKA protein was known and they were docked in the exact same crystallographic coordinates using the rDock program for consistency. For example, to perform the tethered docking studies, five PDB structures (PDB ID: 3NX8, 3OOG, 5BX6, 5BX7, 5N3Q) were used to extract the co-crystallized fragment coordinates. These five structures were chosen due to the identical binding sites of the fragments involved.

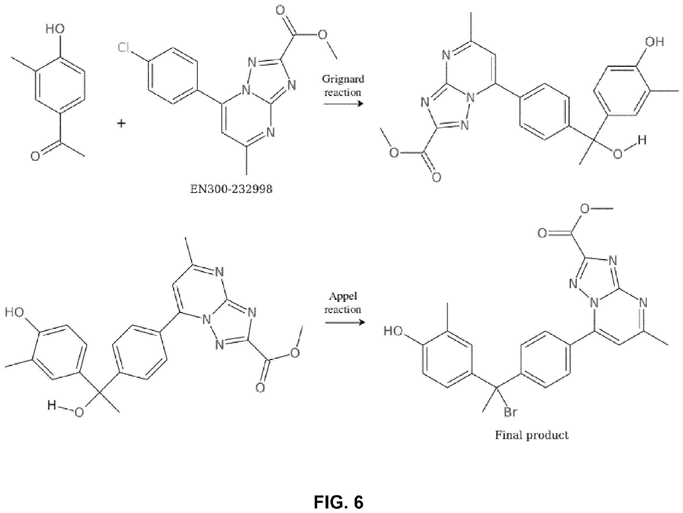

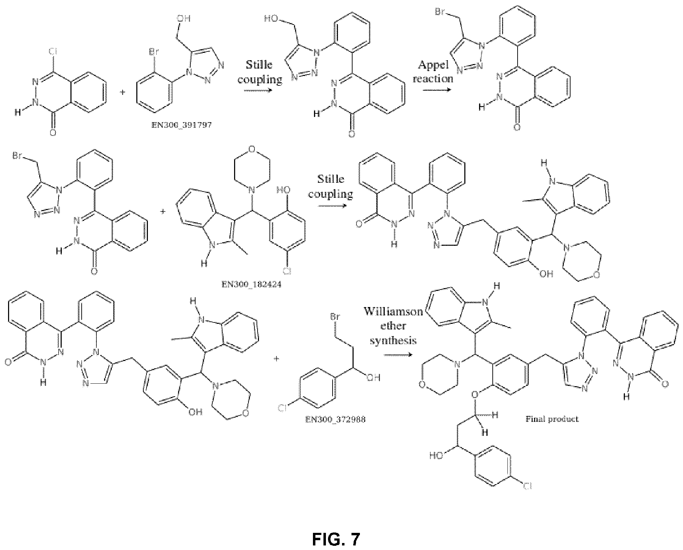

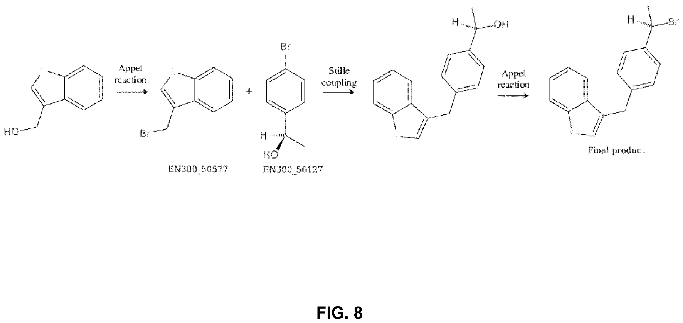

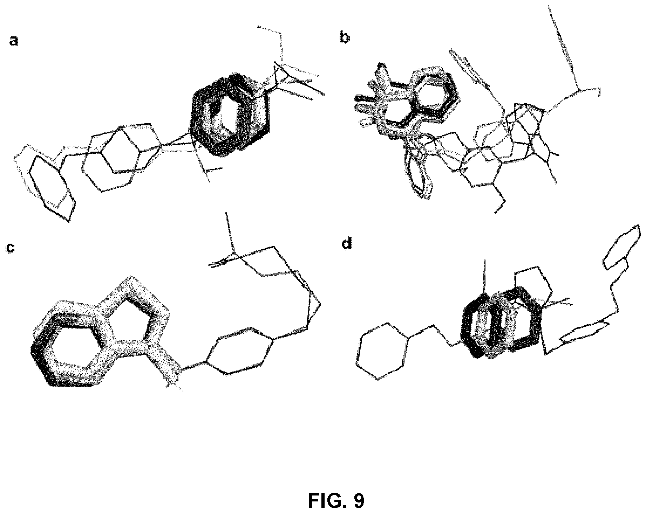

For the tethered docking technique, the coordinates of the scaffold of a chosen crystallographic fragment from literature has been obtained. These coordinates have been used to anchor the scaffold of all generated reaction intermediates and products to the same position within the binding site of the target protein. For untethered docking technique, no seed coordinates are required, and the docking technique is entirely done from scratch using only the information of the binding site in the target protein. This does not guarantee that the successive reaction intermediates will have common anchor points within the binding site. FIGS. 5 through 8, with reference to FIGS. 1 through 4, depict a complete forward synthesis route predicted by Monte Carlo Tree Search (MCTS) search tree with the tethered docking technique for run 1 of various molecular fragments, in accordance with an embodiment of the present disclosure. More specifically, FIG. 5 depicts a complete forward synthesis route predicted by Monte Carlo Tree Search (MCTS) search tree with the tethered docking technique for run 1 of molecular fragment (PDB ID: 3NX8), in accordance with an embodiment of the present disclosure. FIG. 6 depicts the complete forward synthesis route predicted by Monte Carlo Tree Search (MCTS) search tree with the tethered docking technique for run 1 of molecular fragment (PDB ID: 3OOG), in accordance with an embodiment of the present disclosure. FIG. 7 depicts the complete forward synthesis route predicted by Monte Carlo Tree Search (MCTS) search tree with the tethered docking technique for run 1 of molecular fragment (PDB ID: 5BX6), in accordance with an embodiment of the present disclosure. FIG. 8 depicts the complete forward synthesis route predicted by Monte Carlo Tree Search (MCTS) search tree with the tethered docking technique for run 1 of molecular fragment (PDB ID: 5BX7), in accordance with an embodiment of the present disclosure. The Enamine identifiers of the building blocks are also provided in the FIGS. 5 through 8. As mentioned, all examples provided in FIGS. 5 through 8 were obtained from tethered docking experiments. FIG. 9, with reference to FIGS. 1 through 10, depicts a representation of how molecules are anchored to the binding site based on the scaffold present in a crystallographic (molecular) fragment, in an accordance with an embodiment of the present disclosure.

Referring to steps of FIG. 3, at step 208 of the method of the present disclosure, the one or more hardware processors 104 determine a plurality of reaction templates pertaining to the first molecular fragment using a trained reaction template selection model. An expansion and simulation policy (reaction template selection policy) has been used by the system 100 and the method of the present disclosure to decide a suitable reaction template for the starting reactant/first molecular fragment or reaction intermediate (described later), to grow the search tree further. Hence, a template classification model is trained with a downsampled Enamine reactants dataset as input. For any reactant, this trained policy can provide a probability distribution over the possible reaction templates that can be used at that step of the search. In FIGS. 5 through 8, Bromination, Stille Coupling, Appel reaction, Williamson ether synthesis, Grignard reaction etc., are examples of the reaction templates chosen for the expansion and simulation steps of the MCTS search tree process.

With the atomistic molecular graph representation of the reactant molecule, a graph convolution network (GCN) was used to predict the reaction template. The architecture included a single GCN layer followed by a fully connected layer and a final dense layer with softmax activation. The output hidden state from the GCN layer was subject to global mean pooling to get graph-level readout from the model. The downsampled Enamine reactants/molecular fragments dataset was split in a 9: 1: 1 ratio using the stratified split method in scikit-learn. Categorical cross-entropy loss was used to train the model and the parameters were optimized using the Adam optimizer. The initial learning rate was set to 0.005 and the model was trained for 100 epochs with a dropout rate of 0.5 to prevent overfitting. The model was implemented with the Deep Graph Library (DGL) framework using PyTorch as the backend.

At step 210 of the method of the present disclosure, the one or more hardware processors 104 perform, for each reaction template amongst the plurality of reaction templates based on a type of a reaction template, one of following steps to obtain one or more reaction intermediates. The type of the reaction template comprises at least one of a unimolecular reaction template, and a bimolecular reaction template. More specifically, at step 210a of the method of the present disclosure, the one or more hardware processors 104 obtain a reaction intermediate based on the first molecular fragment. In particular, the reaction intermediate is obtained for each reaction template amongst the plurality of reaction templates for the first molecular fragment when (or if) a given reaction template is a unimolecular reaction template. The reaction intermediate is obtained using the RDkit tool, in an embodiment of the present disclosure. If the reaction template chosen is unimolecular, the RDKit RunReactants() function is used to apply the chosen reaction template on the first molecular fragment. For instance, in FIG. 5, the first reaction template chosen is Bromination, which is a unimolecular reaction. So, it was directly applied on the first molecular fragment to obtain the reaction intermediate (with bromine instead of hydroxyl group).

Alternatively, at step 210b of the method of the present disclosure, the one or more hardware processors 104 select a second molecular fragment using the trained molecular fragment selection policy and obtain the reaction intermediate. The trained molecular fragment selection policy is configured to select the second molecular fragment by predicting a property vector of one or more candidate second molecular fragments from at least one fragment library obtained from at least one source (e.g., say a purchasable source as mentioned above).

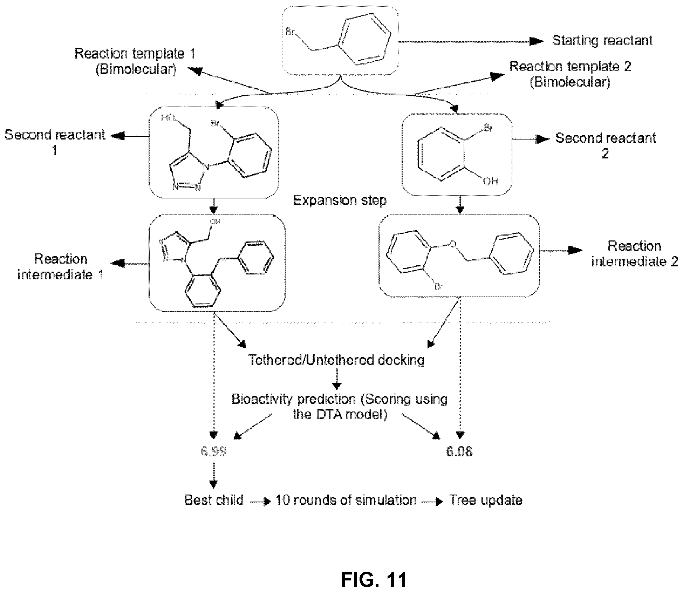

If the reaction template chosen is bimolecular, the trained molecular fragment selection policy is used to predict the second reactant/molecular fragment for completing the chemical reaction. This policy predicts a vector of physicochemical properties specific to the probable second reactant/ molecular fragment. This vector is used to perform a kNN search over the library of purchasable fragments (like Enamine), to obtain the closest second reactant which can be used in the reaction. Once this reactant is identified, the RDKit RunReactants() function is again used to obtain the reaction intermediate. For instance, in FIG. 5, the output of Bromination reaction has been used to run the reaction template selection policy again. It leads to selection of Stille coupling as the next template, which is bimolecular. In this case, the trained molecular fragment selection policy is applied to choose EN300_17995 (a fragment from the Enamine library) as the second reactant, which is further used to complete the reaction. The above step 210b is further better understood by way of following description:
The property vector used to describe the second probable/candidate reactant/molecular fragment comprises of a set of 35 physicochemical properties namely: MaxEStateIndex, MinEStateIndex, MinAbsEStateIndex, QED, MolWt, FpDensityMorgan1, BalabanJ, PEOE VSA10, PEOEVSA11, PEOE VSA6, PEOE VSA7, PEOE VSA8, PEOEVSA9, SMR VSA7, SlogP _VSA3, SlogP _VSA5, EState _VSA2, EState _VSA3, EState_VSA4, EState _VSA5, EState _VSA6, FractionCSP3, MolLogP, Kappa2, PEOE VSA2, SMR VSAS, SMR VSA6, EState_VSA7, Chi4v, SMR_ VSA10, SlogP _VSA4, SlogP _VSA6, EState _VSA8, EState _VSA9, VSA_EState9. A detailed description of each of these 35 properties is available in the RDKit documentation in literature. The trained molecular fragment selection policy is a regression model trained to predict the vector of 35 properties provided above, which provides an approximation of the property space within which the suitable second reactant lies. For instance, in FIG. 11, for identification of second reactant/molecular fragment 1 and second reactant/ molecular fragment 2, the chosen reaction template and the first molecular fragment are provided as inputs to the trained molecular fragment selection policy. This trained model/policy predicts a property vector corresponding to the space where the second reactants/molecular fragment are present.

Further, the second molecular fragment is selected from the fragment library based a comparison of the property vector of the one or more candidate second molecular fragments and an associated property vector of each molecular fragment comprised in the at least one fragment library. Using the property vector obtained above, the second reactant/molecular fragment is predicted through a k-nearest neighbors (kNN) search where Euclidean distance is used as the distance metric to measure similarity between the predicted property vector and property vectors of the fragments present in any fragment library (such as Enamine). In other words, upon comparison of the predicted property vectors, the second reactant/molecular fragment 1 and second reactant/ molecular fragment 2 shown in FIG. 11 were identified through kNN search. These second reactants/ molecular fragments are then reacted with the first molecular fragment using the RDKit RunReactants() function and the chosen molecular template, to obtain reaction intermediates 1 and 2 (child nodes of the search tree). In the present disclosure, the system 100 and the method trained the molecular fragment selection policy with mean squared error (MSE) loss function and the parameters are optimized with the Adam optimizer. The initial learning rate was set to 0.005 and the model was trained for 100 epochs with a dropout rate of 0.5 to prevent overfitting. The model was implemented with the Deep Graph Library (DGL) framework using PyTorch as the backend.

Once the one or more reaction intermediates are obtained, at step 212 of the method of the present disclosure, the one or more hardware processors 104 perform/apply, the at least one docking technique, on the one or more reaction intermediates to obtain a second docking output. In other words, the predicted complex structures are dynamically generated using the rDock program, to compute a reward for simulation (described later). As described in step 204, similar docking technique is applied/performed on the one or more reaction intermediates. Hence, for the sake of brevity, the same is not illustrated.

At step 214 of the method of the present disclosure, the one or more hardware processors 104 compute a second bioactivity value of the one or more reaction intermediates using the DTA model based on the second docking output. As described in step 206, similar bioactivity value is computed for the one or more reaction intermediates. Hence, for the sake of brevity, the same is not illustrated.

At step 216 of the method of the present disclosure, the one or more hardware processors 104 select at least one reaction intermediate amongst one or more reaction intermediates based on the second bioactivity value. In the present disclosure, the at least one reaction intermediate serves as a child node of the first molecular fragment. The reaction intermediate having a highest bioactivity value amongst the one or more reaction intermediates. Below Table 1 illustrates results from the MCTS calculations. The text in bold in Table 1 indicates molecules with the best predicted bioactivity, least similarity to existing actives against PKA, and also shows improvement in terms of the predicted bioactivity to the existing inhibitor.

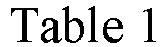

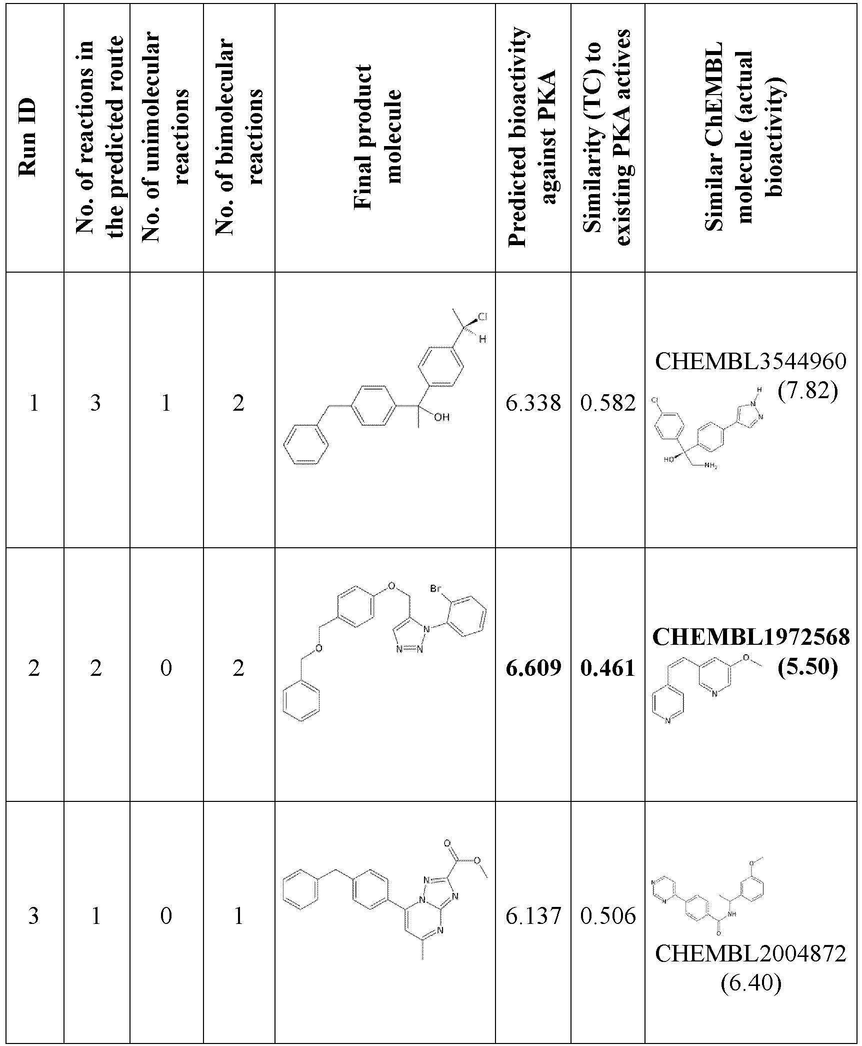

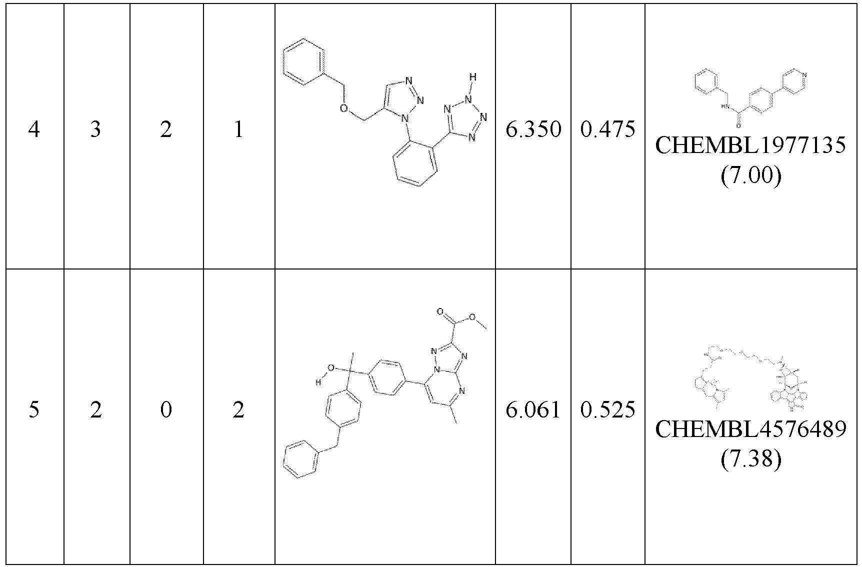

Analysis of the intermolecular interactions observed over the course of the forward synthesis route indicated a steady increase in bioactivity with the growth of the molecule in the binding site (refer FIG. 9). The following binding site residues were found to consistently interact with both existing inhibitors and generated small molecules from this study: Argl9, Gly50, Thr51, Gly52, Val57, Lys168, Glu170, Thr183 and Asp 184. The above step of 216 is better understood by way of following description:

From the expansion step described till step 214, a set of child nodes or reaction intermediates are generated by the MCTS search tree process, along with their predicted bioactivity values from the DTA model. These bioactivity values are used to compute the Q-value or node-level reward based on a tree policy function like the Upper Confidence Bounds (UCB) condition. This Q-value is used to decide the best child node among the set of child nodes obtained and this best child node along with its bioactivity value is updated in the MCTS search tree (also referred to the search tree mentioned above). FIG. 11, with reference to FIGS. 1 through 10, depicts an expansion step from the first molecular fragment for selection of at least one reaction intermediate amongst one or more reaction intermediates, in accordance with an embodiment of the present disclosure. For instance, in FIG. 11, there are two possible child nodes from the first molecular fragment (termed reaction intermediates 1 and 2). Based on the docking and bioactivity-based scoring/ bioactivity values of the child nodes, it is observed that, reaction intermediate 1 is the best child, due to its higher bioactivity value in comparison with the other child node. Hence, reaction intermediate 1 is considered as the best child and populated in the MCTS search tree.

At step 218 of the method of the present disclosure, the one or more hardware processors 104 iteratively perform a simulation on the search tree by repeating the steps of (iv) through (viii), using the child node, to obtain a best child node. The child node is appended to the first molecular fragment along with the first bioactivity value, or the best child node based on a previous iteration, in an embodiment of the present disclosure. The simulation is iteratively performed in step 218 until at least one of (a) a bioactivity value of a selected child node is greater than a predefined bioactivity threshold and (b) a depth of the search tree is greater than a maximum depth allowed. In other words, these serve as termination criteria, wherein the termination condition/criteria include: (a) if the best child bioactivity value is greater than a reward threshold, terminate MCTS, (b) if the MCTS search tree depth is greater than a maximum depth allowed, terminate MCTS, or (c) continue MCTS search tree execution until either of the above conditions is met.

During the update, the rewards and visit counts of the edges and nodes of the search tree, respectively, are updated based on the simulation results from the current state to the root node. The average reward from the simulation is assigned to the node from where the simulation was performed. The state-action pair which produced the maximum average reward post-simulation is selected for the next expansion-simulation-update iteration of MCTS search tree. Here, the root node pertains to the first molecular fragment during the first MCTS iteration. In further iterations, it pertains to the best child obtained post expansion and simulation steps.

In other words, as mentioned in the last step, the root node is the first molecular fragment along with its bioactivity value, followed by the best child node obtained in step 216 along with its bioactivity value. In the subsequent steps, the best child nodes are populated in the search tree to obtain the final MCTS path. For instance, in FIG. 11, the first molecular fragment or root node is labelled the starting reactant and the best child node is reaction intermediate 1. With reaction intermediate 1 as the starting reactant, the simulations will be performed repeatedly until the termination conditions are met.

At step 220 of the method of the present disclosure, the one or more hardware processors 104 generate a molecule with a synthesis route based on a path selected using one or more best child nodes from the search tree. The molecule is used for a drug design and development. The steps 218 and 220 are better understood by way of following description:

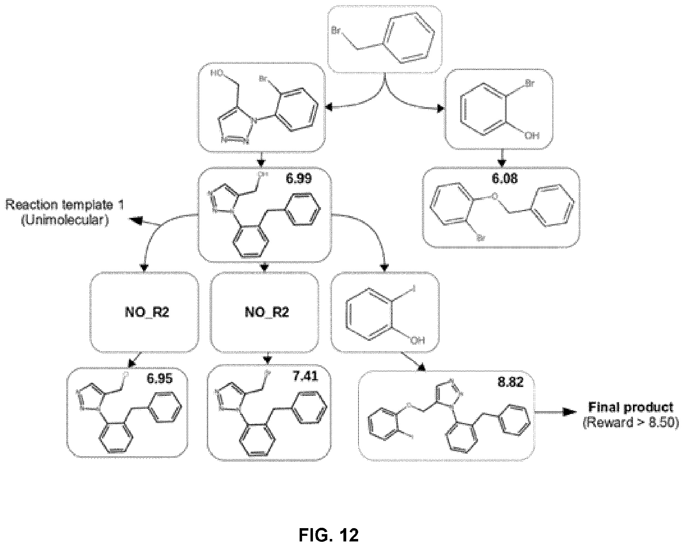

FIG. 12, with reference to FIGS. 1 through 11, depicts a complete MCTS search tree showing a termination of the MCTS search tree process upon generation of a child node with reward greater than the necessary reward threshold (in this case, 8.5), in accordance with an embodiment of the present disclosure. For each iteration of MCTS and for each child node, the simulation was performed 'n' times (e.g., 10 times) and the average reward of the simulations is thresholded to a cut-off bioactivity value to select the child candidate for further expansion. After several iterations of MCTS expansion, simulation and update, the path containing reactants and products with most number of visits and highest average reward is reported, with the final product being the generated molecule of interest (refer FIG. 11). In FIG. 12, the MCTS search tree process was continued only till generation of a best child node with predicted bioactivity greater than 8.5. The best child node so obtained has been labelled as the final product (best child)/molecule, which can be considered as the potential inhibitor against the target protein of interest. The complete formulation of the forward synthesis prediction problem using MCTS is provided as a schematic below (FIG. 2).

Based on the search tree obtained with all the child nodes (reaction intermediates) possible in every MCTS step, the path containing the best child nodes is identified and used as the predicted forward synthesis route for the final best child (final product) by the MCTS search tree process. For instance, in FIG. 12, and FIGS. 5 through 8, the list of reactions shown belong to the identified best forward synthesis route with a steady increase in bioactivity value (e.g., Bromination, Stille coupling, Grignard reaction and Appel reaction are examples of chosen reaction templates, Unimolecular: Bromination and Appel reaction, Bimolecular: Stille coupling and Grignard reaction). These pertain to the final outputs from the MCTS search tree implemented by the system 100 and the method of the present disclosure.

### Performance of generative model with target-specific starting reactants and untethered docking:

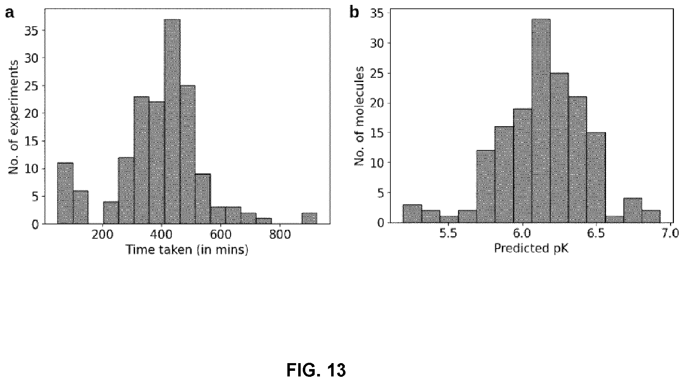

For the untethered docking-based MCTS approach, the Enamine downsampled dataset was mapped to the 537 PKA-binding compounds collected from the ChEMBL database. Inactive molecules (with pK < 6.0) were also considered for the mapping to discover cases where the bioactivity improves even upon starting the search process with reactants enriched in inactive molecules. Histograms showing the time taken for collection of 500 MCTS trajectories and the corresponding distribution of predicted bioactivity values of the final products are provided in FIG. 13. More specifically, FIG. 13, with reference to FIGS. 1 through 12, depicts a histogram illustrating (a) time distribution of the MCTS runs for the building blocks enriched in PKA inactives, wherein an average time taken is 450 mins per run, and (b) distribution of a predicted log-scale bioactivity against PKA for final products obtained from MCTS, in accordance with an embodiment of the present disclosure.

Embodiments of the present disclosure provide systems and methods that are generic in nature and can be applied to any target protein of interest. The method can consider identified fragments (from fragment screening) from existing crystal structures as input and ensure preservation of the fragment throughout the molecule generation process. Tethered docking technique as implemented by the system and method of the present disclosure ensures that the position of the initial fragment is fixed. A structure-based reward was used to retain the target specificity and identify the appropriate fragment from the fragment library during the search in the MCTS search tree process. The method of the present disclosure can also perform de novo molecule generation for a target protein based on the proteins binding site, given a library of suitable starting reactants/molecular fragments. Based on the utilization of a structure-based reward and adding molecular fragments from a fragment library, the method is one of kind where the forward synthesis can be a multi-step process. The MCTS method of the present disclosure has been used for forward synthesis route prediction. The method of the present disclosure provides a structural perspective of the evolution of the molecule at the target binding site, through on-the-fly docking of the molecule using the RxDock program (docking technique). Based on the trained reactant/molecular fragment selection model/policy using bimolecular reactions, second reactant/molecular fragment was chosen/selected from the enamine library. The trained model/policy focused on the relevant chemical space for the second reactant/molecular fragment, making the search process much faster compared to existing methods.

The method and system of the present disclosure use both domain knowledge and various algorithms to navigate the large space of available starting reactants/molecular fragments for synthesis of small molecules. The method also provides on-the-fly structural perspective and validation of the interactions of generated reaction intermediates and the final product through docking. The binding affinity obtained from docking calculations is used to guide the MCTS search tree process.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method comprising:
(i) selecting, via one or more hardware processors, a first molecular fragment from a plurality of molecular fragments comprised in a fragment library (202);
(ii) performing, at least one docking technique via the one or more hardware processors, on the first molecular fragment to obtain a first docking output (204);
(iii) computing, via the one or more hardware processors, a first bioactivity value of the first molecular fragment using a drug-target affinity (DTA) model based on the first docking output (206), wherein a search tree is populated with the first bioactivity value for the first molecular fragment;
(iv) determining, via the one or more hardware processors, a plurality of reaction templates pertaining to the first molecular fragment using a trained reaction template selection model (208);
(v) performing, via the one or more hardware processors, for each reaction template amongst the plurality of reaction templates based on a type of a reaction template (210), one of:
obtaining a reaction intermediate based on the first molecular fragment (210a); and
selecting a second molecular fragment using a trained molecular fragment selection policy and obtaining the reaction intermediate (210b), to obtain one or more reaction intermediates;
(vi) performing, the at least one docking technique via the one or more hardware processors, on the one or more reaction intermediates to obtain a second docking output (212);
(vii) computing, via the one or more hardware processors, a second bioactivity value of the one or more reaction intermediates using the DTA model based on the second docking output (214);
(viii) selecting, via the one or more hardware processors, at least one reaction intermediate amongst one or more reaction intermediates based on the second bioactivity value (216), wherein the at least one reaction intermediate serves as a child node of the first molecular fragment;
(ix) iteratively performing a simulation on the search tree by repeating the steps of (iv) through (viii), using the child node, to obtain a best child node (218), wherein the child node is appended to the first molecular fragment along with the first bioactivity value, or the best child node based on a previous iteration,
until at least one of (a) a bioactivity value of a selected child node is greater than a predefined bioactivity threshold and (b) a depth of the search tree is greater than a maximum depth allowed; and
(x) generating a molecule with a synthesis route based on a path selected using one or more best child nodes from the search tree (220), wherein the molecule is used for a drug design and development.

2. The processor implemented method as claimed in claim 1, wherein the at least one docking technique is a tethered docking technique or an untethered docking technique.

3. The processor implemented method as claimed in claim 2, wherein the at least one docking technique is a tethered docking technique when the first molecular fragment is selected from a result obtained from a crystallographic screening.

4. The processor implemented method as claimed in claim 2, wherein the at least one docking technique is an untethered docking technique when the first molecular fragment is randomly selected.

5. The processor implemented method as claimed in claim 1, wherein the at least one reaction intermediate comprises a highest bioactivity value,
wherein the trained molecular fragment selection policy is configured to select the second molecular fragment by:
predicting a property vector of one or more candidate second molecular fragments from at least one fragment library obtained from at least one source; and
selecting the second molecular fragment from the fragment library based a comparison of the property vector of the one or more candidate second molecular fragments and an associated property vector of each molecular fragment comprised in the at least one fragment library, and
wherein the type of the reaction template comprises at least one of a unimolecular reaction template, and a bimolecular reaction template.

6. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
(i) select a first molecular fragment from a plurality of molecular fragments comprised in a fragment library;
(ii) perform, at least one docking technique, on the first molecular fragment to obtain a first docking output;
(iii) compute a first bioactivity value of the first molecular fragment using a drug-target affinity (DTA) model based on the first docking output, wherein a search tree is populated with the first bioactivity value for the first molecular fragment;
(iv) determine a plurality of reaction templates pertaining to the first molecular fragment using a trained reaction template selection model;
(v) perform, via the one or more hardware processors, for each reaction template amongst the plurality of reaction templates based on a type of a reaction template, one of:
obtaining a reaction intermediate based on the first molecular fragment; and
selecting a second molecular fragment using a trained molecular fragment selection policy and obtaining the reaction intermediate,
to obtain one or more reaction intermediates;
(vi) perform, the at least one docking technique, on the one or more reaction intermediates to obtain a second docking output;
(vii) compute a second bioactivity value of the one or more reaction intermediates using the DTA model based on the second docking output;
(viii) select at least one reaction intermediate amongst one or more reaction intermediates based on the second bioactivity value, wherein the at least one reaction intermediate serves as a child node of the first molecular fragment;
(ix) iteratively perform a simulation on the search tree by repeating the steps of (iv) through (viii), using the child node, to obtain a best child node, wherein the child node is appended to the first molecular fragment along with the first bioactivity value, or the best child node based on a previous iteration,
until at least one of (a) a bioactivity value of a selected child node is greater than a predefined bioactivity threshold and (b) a depth of the search tree is greater than a maximum depth allowed; and
(x) generating a molecule with a synthesis route based on a path selected using one or more best child nodes from the search tree, wherein the molecule is used for a drug design and development (220).

7. The system as claimed in claim 6, wherein the at least one docking technique is a tethered docking technique or an untethered docking technique.

8. The system as claimed in claim 7, wherein the at least one docking technique is a tethered docking technique when the first molecular fragment is selected from a result obtained from a crystallographic screening.

9. The system as claimed in claim 7, wherein the at least one docking technique is an untethered docking technique when the first molecular fragment is randomly selected.

10. The system as claimed in claim 6, wherein the at least one reaction intermediate comprises a highest bioactivity value,
wherein the trained molecular fragment selection policy is configured to select the second molecular fragment by:
predicting a property vector of one or more candidate second molecular fragments from at least one fragment library obtained from at least one source; and
selecting the second molecular fragment from the fragment library based a comparison of the property vector of the one or more candidate second molecular fragments and an associated property vector of each molecular fragment comprised in the at least one fragment library, and
wherein the type of the reaction template comprises at least one of a unimolecular reaction template, and a bimolecular reaction template.

11. One or more non-transitory machine-readable information storage mediums comprising one or more instructions which when executed by one or more hardware processors cause:
(i) selecting, a first molecular fragment from a plurality of molecular fragments comprised in a fragment library;
(ii) performing, at least one docking technique, on the first molecular fragment to obtain a first docking output;
(iii) computing a first bioactivity value of the first molecular fragment using a drug-target affinity (DTA) model based on the first docking output, wherein a search tree is populated with the first bioactivity value for the first molecular fragment;
(iv) determining a plurality of reaction templates pertaining to the first molecular fragment using a trained reaction template selection model;
(v) performing for each reaction template amongst the plurality of reaction templates based on a type of a reaction template , one of:
obtaining a reaction intermediate based on the first molecular fragment; and
selecting a second molecular fragment using a trained molecular fragment selection policy and obtaining the reaction intermediate , to obtain one or more reaction intermediates;
(vi) performing, the at least one docking technique, on the one or more reaction intermediates to obtain a second docking output;
(vii) computing a second bioactivity value of the one or more reaction intermediates using the DTA model based on the second docking output;
(viii) selecting at least one reaction intermediate amongst one or more reaction intermediates based on the second bioactivity value , wherein the at least one reaction intermediate serves as a child node of the first molecular fragment;
(ix) iteratively performing a simulation on the search tree by repeating the steps of (iv) through (viii), using the child node, to obtain a best child node, wherein the child node is appended to the first molecular fragment along with the first bioactivity value, or the best child node based on a previous iteration,
until at least one of (a) a bioactivity value of a selected child node is greater than a predefined bioactivity threshold and (b) a depth of the search tree is greater than a maximum depth allowed; and
(x) generating a molecule with a synthesis route based on a path selected using one or more best child nodes from the search tree, wherein the molecule is used for a drug design and development.

12. The one or more non-transitory machine-readable information storage mediums as claimed in claim 11, wherein the at least one docking technique is a tethered docking technique or an untethered docking technique.

13. The one or more non-transitory machine-readable information storage mediums as claimed in claim 12, wherein the at least one docking technique is a tethered docking technique when the first molecular fragment is selected from a result obtained from a crystallographic screening.

14. The one or more non-transitory machine-readable information storage mediums as claimed in claim 12, wherein the at least one docking technique is an untethered docking technique when the first molecular fragment is randomly selected.

15. The one or more non-transitory machine-readable information storage mediums as claimed in claim 11, wherein the at least one reaction intermediate comprises a highest bioactivity value,
wherein the trained molecular fragment selection policy is configured to select the second molecular fragment by:
predicting a property vector of one or more candidate second molecular fragments from at least one fragment library obtained from at least one source; and
selecting the second molecular fragment from the fragment library based a comparison of the property vector of the one or more candidate second molecular fragments and an associated property vector of each molecular fragment comprised in the at least one fragment library, and
wherein the type of the reaction template comprises at least one of a unimolecular reaction template, and a bimolecular reaction template.
